# EUROPÄISCHE PATENTANMELDUNG

(11) **EP 0 911 792 A2**
(43) Veröffentlichungstag der Anmeldung: **28.04.1999**
(21) Anmeldenummer: 98117971.6
(22) Anmeldetag: 22.09.1998
(51) Int. Cl.: G09G 3/36

(54) **Verfahren für die Bilderzeugung auf einem Farbbildschirm und ein dazu geeigneter Farbbildschirm**

(30) Priorität: 22.10.1997 DE 19746576
(71) Anmelder: Carl Zeiss, 89518 Heidenheim (Brenz) (DE); CARL-ZEISS-STIFTUNG, trading as CARL ZEISS, 89518 Heidenheim (DE)
(72) Erfinder: Wesemann, Wolfgang, 50996 Köln (DE)

(57) **Zusammenfassung**

Die Erfindung betrifft ein Verfahren für die Bilderzeugung auf einem Farbbildschirm mit einer Ansteuerungseinrichtung, wobei jedem Pixel drei beieinander angeordnete, unterschiedliche Farben emmitierenden Farbbereiche zugeordnet sind und die Pixel in Zeilen und/oder Spalten angeordnet sind.

Erfindungsgemäß wird durch die Ansteuerungseinrichtung des Farbbildschirms ein sichtbarer Bildpunkt auf dem Farbbildschirm durch drei nebeneinander liegende Farbbereiche unterschiedlicher, nebeneinander liegender Pixel erzeugt wird.

In einem weiteren erfindungsgemäßen Verfahren werden ein oder mehrere darzustellende Bildbereiche aus mehr als einem Pixel gebildet, wobei die darzustellenden Bildbereiche einen Schwerpunkt der Lichtverteilung besitzen, und für eine Bildbereichsverschiebung auf dem Bildschirm werden die verschiedenen Pixel so durch die Ansteuerungseinrichtung angesteuert, daß die sich eine vorgegebene Schwerpunktverschiebung des darzustellenden Bildbereichs ergibt.

Bei dem erfindungsgemäßen Farbbildschirm mit einer Ansteuerungseinrichtung sind einem Pixel drei beieinander angeordnete, unterschiedliche Farben emmitierenden Farbbereiche zugeordnet und die Pixel sind in Zeilen und/oder Spalten angeordnet.

Erfindungsgemäß ist die Ansteuerungseinrichtung des Farbbildschirms so aufgebaut, daß ein sichtbarer Bildpunkt auf dem Farbbildschirm durch drei nebeneinander liegende Farbbereiche unterschiedlicher, nebeneinander liegender Pixel erzeugt wird und/oder ein oder mehrere darzustellende Bildbereiche aus mehr als einem Pixel gebildet werden, wobei die darzustellenden Bildbereiche einen Schwerpunkt der Lichtverteilung besitzen, und für eine Bildbereichsverschiebung eine Schwerpunktverschiebung des darzustellenden Bildbereichs erfolgt.

## Beschreibung

Die Erfindung betrifft Verfahren für die Bilderzeugung auf einem Farbbildschirm mit einer Ansteuerungseinrichtung, wobei jedem Pixel drei beieinander angeordnete, unterschiedliche Farben emmitierenden Farbbereiche zugeordnet sind und die Pixel in Zeilen und/oder Spalten angeordnet sind und einen zur Durchführung der Verfahren geeigneten Farbbildschirm.

Bei den derzeit üblichen Farbbildschirmen werden die Bilder aus einzelnen Pixeln aufgebaut, die in einer Matrix angeordnet sind. Diese Farbbildschirme werden auch als Matrix-Displays bezeichnet.

Unter einem Pixel versteht man einen durch seine Zeilen- und Spaltennummer definierten Punkt auf einem Bildschirm, der bunt oder unbunt leuchten kann. Jedes Pixel setzt sich bei einem Farbmonitor aus drei farbigen Pixelteilen (Farbbereichen) zusammen, die in der Regel in den drei Grundfarben rot, grün und blau leuchten. Die farbigen Pixelteile sind entweder nebeneinanderliegende Farbrechtecke oder in einer hexagonalen Anordnung liegende Farbkreise.

Ein derartiger Farbbildschirm kann für Sehtestgeräte verwendet werden, wobei häufig die Auflösung ein beschränkendes Element ist.

Eine Vielzahl unterschiedlicher Hilfsmittel bzw. Geräte wird heute insbesondere bei Augenärzten und Augenoptikern zur Brillenglasbestimmung und zur Sehprüfung verwendet. Dazu gehören: Sehprobentafeln, Sehzeichenprojektoren, Durchlichtsehprüfgeräte und Computersehprüfgeräte.

Mit einer gedruckten Sehprobentafel kann man natürlich nur die Prüfungen durchführen, für die sie entworfen wurde.

Mit Sehzeichenprojektoren und Durchlichtsehprüfgeräten können Sehzeichen gewechselt werden. Bei Verwendung entsprechender Zusatzeinrichtungen ist auch die Möglichkeit einer binokularen Funktionsprüfung gegeben.

Eine Sehprüfung mit dem Computer ermöglicht darüber hinaus die Prüfung von Sehleistungsfunktionen, für die man bisher mehrere Spezialsehprobentafeln oder speziell ausgerüstete Sehprüfgeräte benötigte.

Bei der Sehprüfung verfolgt man das Ziel, Wahrnehmungsschwellen zu bestimmen, die als Maß für die Leistungsfähigkeit des visuellen Systems angesehen werden können. Dazu werden mit Sehtestgeräten Sehzeichen in abgestuften Reizstärken dargeboten. Die Art der Sehzeichen richtet sich dabei nach der zu testenden Sehfunktion. Zur Prüfung der Sehschärfe verwendet man z.B. Buchstaben in abgestuften Größen.

Die Forderungen an Sehprüfgeräte, mit denen Wahrnehmungsschwellen gemessen werden sollen, unterscheiden sich von den anderen Anforderungen. Zur Prüfung des räumlichen Sehens (Stereosehens) verwendet man z.B. synthetische Stereobilder mit unterschiedlichen Tiefenunterschieden, bei denen einzelne Bildbestandteile vor der Betrachtungsebene zu schweben scheinen. Das Ergebnis dieser Prüfungen ist die Größe des kleinsten, gerade noch erkennbaren Buchstabens bzw. die Größe des kleinsten, gerade noch sichtbaren räumlichen Tiefenunterschieds. Diese Werte kennzeichnen physiologischen Wahrnehmungsschwellen des untersuchten Auges.

Um physiologische Wahrnehmungsschwellen messen zu können, müssen Sehzeichen dargestellt werden können, deren Reizstärken von der Größenordnung der Wahrnehmungsschwelle sind. Eine genaue Messung der Wahrnehmungsschwelle erfordert zudem, daß
a) die Schrittweite, mit der die Reizstärke verändert werden kann, möglichst klein ist und daß
b) auch Reizstärken dargestellt werden können, die kleiner als der sinnesphysiologische Wahrnehmungsschwellenwert sind.

Augengesunde Menschen verfügen über zwei Augen und dadurch über die Fähigkeit zum Binokularsehen, welche eine Grundvorraussetzung zur räumlichen Tiefenwahrnehmung ist.

Beim Binokularsehen unterscheidet man drei Stufen:
a) das beidäugige Simultansehen ohne Fusion (Fusion = Fähigkeit zur Verschmelzung der beiden monokularen Seheindrücke im visuellen Cortex),
b) das beidäugige Simultansehen mit Fusion, sowie
c) das beidäugige Simultansehen mit Stereosehen (Stereosehen gleichbedeutend mit Stereopsis bzw. querdisparatem Raumsehen).

Eine notwendige Voraussetzung für das Stereosehen ist die sensorische Verschmelzung der Bildeindrücke beider Augen im Gehirn. Durch das Stereosehen entsteht ein "lebendiger" räumlicher Seheindruck.

Das Stereosehen beruht auf dem Umstand, daß die beiden Augen des Menschen die Umwelt aus zwei verschiedenen Blickrichtungen betrachten. Dadurch entstehen bei der Betrachtung eines 3-dimensionalen Objekts in beiden Augen Netzhautbilder, die sich nicht exakt gleichen, sondern - aufgrund der unterschiedlichen Blickrichtungen - horizontal versetzte Komponenten aufweisen.

Wenn z.B. ein Augenpaar einen Objektpunkt O im Abstand a fixiert, wird dieser Objektpunkt in die Fovea beider Augen abgebildet. Ein vor diesem Objektpunkt gelegener zweiter Punkt P wird hingegen nicht mehr auf korrespondierende Netzhautstellen abgebildet, sondern im linken Auge rechts von der Fovea und im rechten Auge links von der Fovea. Der horizontale Versatz der beiden Bilder von P zueinander wird als "Querdisparation" bezeichnet. Als Maß für die Größe der Querdisparation dient der "Stereowinkel".

Der genaue Zusammenhang zwischen Stereowinkel, Objektentfernungen und Pupillenabstand ist in dem Handbuch für Augenoptik, herausgegeben von der Firma Carl Zeiss, 73447 Oberkochen, neu bearbeitet von Dr. Helmut Goersch, 3. Auflage 1987 auf den Seiten 87 bis 92 beschrieben.

Der kleinste Stereowinkel, der zum Stereosehen führt, heißt "Stereogrenzwinkel". Er ist ein Maß für die Wahrnehmungsschwelle des Stereosehens und beträgt für das menschliche Tagessehen etwa 10 Winkelsekunden. Wenn man diese Wahrnehmungsschwelle genau bestimmen will, braucht man Sehprüfgeräte, die räumliche Sehzeichen mit Stereowinkeln kleiner als 10 Winkelsekunden erzeugen können.

Die Fähigkeit zum Stereosehen muß unterschieden werden von der Fähigkeit zur monokularen Abschätzung räumlicher Tiefe. Über das Stereosehen verfügen nur Menschen mit einem intakten Binokularsehen, während der Abstand eines Objektes auch von Menschen abgeschätzt werden kann, die diese Fähigkeit nicht aufweisen, wie z.B. funktionell Einäugige. Solche Personen können räumliche Tiefe über zusätzliche visuelle Informationen wie z.B. die Netzhautbildgröße, Objektüberschneidungen, Licht, Glanz, Schatten und die Bewegungsparallaxe abschätzen.

Das Anforderungsprofil für ein universelles Sehprüfgerät umfaßt die folgenden Punkte:

Die unterschiedlichen Arten von Sehzeichen - Buchstaben, Ziffern, Landoltringe und das Snellen-E - um nur die wichtigsten zu nennen, müssen von einem universellen Sehprüfgerät darstellbar sein. Darstellbar in unterschiedlichen Größen und Zusammensetzungen für einen Bereich der Visusanforderungen zwischen 0,05 und 2,0. Zusätzlich sollten Symbole, also z.B. Rechtecke, Kreise, Kreuze und Halbfelder für spezielle Sehprüfungen verfügbar sein.

Selbstverständlich sollten alle Sehzeichen auch in Kontrast und Farbe veränderbar sein, damit farbige Sehtests und eine Prüfung des Sehens bei niedrigen Kontrasten durchgeführt werden können. Des weiteren ist es vorteilhaft, wenn auch bewegte Sehzeichen darstellbar sind.

Ergänzend sollten graphische Darstellungen möglich sein und, um die Aufmerksamkeit von Kindern bei der Sehprüfung zu erhöhen, ist die Darstellung von Bildern wünschenswert. Grundsätzlich eröffnet die softwaregesteuerte Bilderzeugung darüber hinaus heute noch nicht genutzte bzw. nicht angedachte Möglichkeiten der Sehprüfung und Sehtestung.

Das Prüffeld, in dem die Sehzeichen dargeboten werden, muß natürlich auch verschiedenen Anforderungen genügen. Es muß sich vor allem in der Lichtqualität so nah wie möglich an die natürlichen Gegebenheiten anpassen, d.h. es muß flimmerfrei sein und eine sonnenlichtähnliche Lichtfarbe aufweisen. Das Prüffeld sollte auch, um neue Dimensionen der Sehprüfung zu eröffnen, in Farbe und Helligkeit variierbar sein.

Die Darbietung der Sehzeichen muß variabel sein, d.h. gruppenweise oder einzeln, mit oder ohne zufallsgesteuerter Variation.

Ein weiterer wichtiger Punkt ist die Prüfung des Binokularsehens und des Stereosehens. Hierzu muß durch eine technische Vorrichtung die Möglichkeit gegeben sein, beiden Augen unterschiedliche Seheindrücke darzubieten. Die beste Darbietungsmethode ist anerkanntermaßen eine zeitgleiche getrennte Darbietung der Sehzeichen für das rechte und das linke Auge im gleichen Sehfeld, kurz: "durch simultane Trennung der Seheindrücke". Soweit die Anforderungen, die an ein universell einsetzbares Sehprüfgerät zu stellen sind.

Neue Verfahren zur Verbesserung der Darstellung der Sehzeichen unterliegen zudem der Nebenbedingung, daß an dem bekannten (Farb-) Bildschirm möglichst wenig verändert werden muß, da es sich um eine Spezialanwendung der Bildschirme handelt und man für diese Spezialanwendung auf käufliche Farbbildschirme, welche in großen Stückzahlen hergestellt werden, zurückgreifen möchte, damit die Sehtestgeräte nicht unnötig teuer sein müssen.

Die heute bekannten Testverfahren zur Prüfung des Stereosehens sind moderne Varianten des schon im vorigen Jahrhundert vorgestellten Wheatstone'schen Stereoskops. Bei dieser Darbietungsart werden dem linken und dem rechten Auge über eine geeignete Bildtrennereinrichtung unterschiedliche zweidimensionale Bilder gezeigt. Diese zwei Bilder zeigen das räumliche Objekt so, wie es von den beiden Augen aus den unterschiedlichen Blickrichtungen gesehen würde. Die beiden Teilbilder weisen querdisparat verschobene Bildanteile auf. Bei der sensorischen Verschmelzung der beiden Teilbilder rekonstruiert das visuelle System die Räumlichkeit des dargestellten Objekts.

Bis vor einiger Zeit wurde als kommerzielle Stereotests hauptsächlich gedruckte Testbilder verwendet, bei denen die querdisparate Versetzung der räumlichen Bildanteile drucktechnisch realisiert wird. In den letzten Jahren wurden Sehtestverfahren vorgestellt, bei denen das Testbild auf einem Monitor oder einem LCD-Display angezeigt wird. Bei diesen Bildschirmverfahren gibt es aber das Problem, daß die Querdisparation nicht - wie bei drucktechnischen Verfahren - kontinuierlich, sondern nur in diskreten Schritten in Einheiten der Pixelbreite verändert werden kann.

Die Schrittweite, mit der die Querdisparation verändert werden kann, ergibt sich aus der Anzahl der auf dem Monitor in jeder Zeile darstellbaren Bildpunkte. Wird z.B. ein LCD-Display mit einer Pixelbreite von 0,33 mm verwendet, so ist der kleinste realisierbare Stereowinkel und die Schrittweite aus einer Entfernung von 5 m gleich arctan(0,33mm/5000mm) = 13,6 Winkelsekunden. Es ist offensichtlich, daß dies nicht ausreicht, um einen Stereogrenzwinkel von 10 Winkelsekunden meßtechnisch genau zu erfassen. Deshalb kann mit derzeit kommerziell erhältlichen Monitoren keine genaue Bestimmung des Stereogrenzwinkels durchgeführt werden. Aus diesem Grund müssen neue Verfahren entwickelt werden, mit denen die Positioniergenauigkeit von Bildbestandteilen auf einem Matrix-Display verbessert werden kann.

Es ist die Aufgabe der Erfindung, Verfahren für die Bilderzeugung auf einem an sich bekannten Farbbildschirm zu entwickeln, welche erlauben, Bilder genauer zu positionieren als bisher.

Diese Aufgabe wird durch ein Verfahren gemäß dem kennzeichnenden Teil des ersten oder zweiten Patentanspruchs sowie durch einen Farbbildschirm nach dem kennzeichnenden Teil des achten Patentanspruchs gelöst.

Die erfindungsgemäßen Verfahren verbessern die horizontale Positioniergenauigkeit von Bildbereichen auf einem Farbbildschirm mit einer Ansteuerungseinrichtung. Die Verfahren eignen sich besonders für die Erzeugung räumlicher Bilder bzw. zur Prüfung des Stereosehens.

Die Erfindung betrifft somit Verfahren für die Bilderzeugung auf einem Farbbildschirm mit einer Ansteuerungseinrichtung, wobei jedem Pixel drei beieinander angeordnete, unterschiedliche Farben emmitierende Farbbereiche zugeordnet sind und die Pixel in Zeilen und/oder Spalten angeordnet sind.

Erfindungsgemäß wird durch die Ansteuerungseinrichtung des Farbbildschirms ein sichtbarer Bildpunkt auf dem Farbbildschirm durch drei nebeneinander liegende Farbbereiche unterschiedlicher, nebeneinander liegender Pixel erzeugt (Dabei wird die feste Zuordnung der drei Farbbereiche zu einem bestimmten, räumlich fixierten Pixel aufgehoben zugunsten einer variablen Zuordnung der Farbbereiche zu einem Pixel, dessen Lage durch die Auswahl der das Pixel ausmachenden Farbbereiche festgelegt ist.), oder ein oder mehrere darzustellende Bildbereiche werden aus mehr als einem Pixel gebildet, wobei die darzustellenden Bildbereiche einen Schwerpunkt der Lichtverteilung besitzen, und für eine Bildbereichsverschiebung auf dem Bildschirm die Farbbereiche der Pixel so durch die Ansteuerungseinrichtung angesteuert werden, daß sich die vorgegebene Schwerpunktverschiebung des darzustellenden Bildbereichs ergibt.

Der zur Durchführung des erfindungsgemäßen Verfahrens benötigte Farbbildschirm zeichnet sich erfindungsgemäß dadurch aus, daß die Ansteuerungseinrichtung des Farbbildschirms so aufgebaut ist, daß
1) ein Bildpunkt auf dem Farbbildschirm durch drei nebeneinander liegende Farbbereiche unterschiedlicher, nebeneinander liegender Pixel erzeugt wird
   und/oder
2) ein oder mehrere darzustellende unbunte oder farbige Bildbereiche aus mehr als einem Pixel gebildet werden, wobei die darzustellenden Bildbereiche einen Schwerpunkt der Lichtverteilung besitzen, und für eine Bildbereichsverschiebung eine Schwerpunktverschiebung des darzustellenden Bildbereichs erfolgt.

Die geringste Verschiebung eines Bildes, die mit einem Matrix-Display erzeugt werden kann, ist nach dem Stand der Technik durch die Breite eines Pixels gegeben. Bei dem derzeit im POLATEST E verwendeten TFT-Displays beträgt die Breite eines Pixels 0,33 mm. Daraus ergibt sich aus 5 m ein minimaler Stereowinkel von 13,6 Winkelsekunden.

Mit beiden erfindungsgemäßen Verfahren wird die horizontale Bildauflösung verbessert. Die erfindungsgemäßen Verfahren eignen sich insbesondere für ein Sehtestgerät. Sie zeichnen sich dadurch aus, daß durch eine Ansteuerungseinrichtung des Farbbildschirms ein oder mehrere Bildbereiche auf dem Bildschirm genauer positioniert werden können als bisher. Die horizontale Positionierung von Bildbereichen erfolgt beim ersten Verfahren mit einer Genauigkeit von 1/3-Pixel durch die Ansteuerung der Farbbereiche unterschiedlicher, nebeneinanderliegender Pixel und beim zweiten Verfahren mit einer beliebigen Genauigkeit durch eine Verschiebung des Lichtschwerpunktes der in den Bildbereichen vorhandenen Lichtverteilung.

Damit muß aber bei einem bekannten Farbbildschirm lediglich die Ansteuerungseinrichtung des Farbbildschirms verändert werden, damit die erfindungsgemäßen Verfahren eingesetzt werden können. Die Anpassung der Ansteuerungseinrichtung kann dabei soft- und/oder hardwaremäßig erfolgen,je nach dem Aufbau der jeweiligen Ansteuerungseinrichtung.

Durch die Verwendung der erfindungsgemäßen Verfahren erhält man somit einen Farbbildschirm mit einer höheren Positioniergenauigkeit, der insbesondere zur Messung der Wahrnehmungsschwelle des Stereosehens vorteilhaft eingesetzt werden kann.

Der Farbbildschirm ist vorzugsweise ein TFT-Display, über dem ein Streifenpolarisator angebracht ist, damit man mittels einer vom Probanden zu tragenden Polarisationsbrille, bei welcher den beiden Augen jeweils eine zum anderen Auge senkrecht stehende Polarisationsachse angeboten wird, die Stereowahrnehmung testen kann. Es können aber auch andere Displays und Trennerverfahren eingesetzt werden, die es erlauben, dem rechten und linken Auge verschiedene Teilbilder darzubieten.

Alle in der DE 41 15 145 und der DE 94 13 371 gemachten Angaben für das dort beschriebene Sehtestgerät gelten sinngemäß auch für das erfindungsgemäße Sehtestgerät, wobei die gemachten Angaben auch sinngemäß für den erfindungsgemäßen Farbbildschirm gelten.

Die Erfindung wird im folgenden anhand eines Ausführungsbeispiels, unter Bezugnahme auf die beiliegenden Figuren, näher erläutert, wobei das nachfolgende Beispiel keinen abschließenden Charakter für die Erfindung hat und weitere vorteilhafte Ausgestaltungen der Erfindung beinhaltet.

Es sind zu sehen in
- Figur 1: eine Detailansicht eines TFT-Displays;
- Figur 2: eine Teilansicht eines TFT-Displays; und
- Figur 3: die Lichtintensität entlang eines Zeilenbereichs, welche für das rechte Auge eines Beobachters bestimmt ist gemäß dem zweiten erfindungsgemäßen Verfahren; und
- Figur 4: die Lichtintensität entlang eines Zeilenbereichs, welche für das linke Auge eines Beobachters bestimmt ist (gemäß dem zweiten erfindungsgemäßen Verfahren).

Die erfindungsgemäßen Verfahren werden im folgenden anhand des Sehprüfgerätes POLATEST E beschrieben, zu welchem es die Schutzrechte DE 41 15 145 und der DE 94 13 371 gibt und mit welchem möglichst viele Sehleistungsprüfungen mit einem einzigen Sehprüfgerät durchgeführt werden können.

Ein derart universell einsetzbares Sehprüfgerät erfüllt sowohl alle Anforderungen der Brillenglasbestimmung und ist auch bei der Sehprüfung und dem Screening der Sehfunktionen einsetzbar. Dieses universelle Sehprüfgerät bietet natürlich grundsätzlich die Möglichkeit, neue Methoden der Sehleistungsprüfung aufzunehmen.

Die in der DE 94 13 371 gefundene Lösung - unter dem Handelsnamen Sehprüfgerät POLATEST E bekannt - wird im weiteren in zwei Schritten dargestellt und erfüllt alle oben gemachten Anforderungen.

Für die Darbietung von Sehzeichen, bei denen keine Trennung der Seheindrücke für das rechte und das linke Auge erfolgen soll, erfüllen bezüglich der Sehzeichendarstellung Farbflachbildschirme, wie sie bei tragbaren Computern Verwendung finden, alle vorher genannten Anforderungen.

Die Sehzeichen werden elektronisch in einem Graphikformat von 640 x 480 Pixeln erzeugt entsprechend dem heutigen technischen Stand von Farbflachbildschirmen. Dies ermöglicht die Darstellung von Sehzeichen bis zu einer Visusanforderung von 2,0 bei einer Prüfentfernung von mehr als 4 m. Auch runde Sehzeichen sind gut darstellbar, wenn man Rundungsalgorythmen verwendet.

Die hohen Anforderungen der Sehprüfung an das Prüffeld - Lichtqualität möglichst den natürlichen Gegebenheiten entsprechend und flimmerfrei - lassen sich mit neuartigen Kompaktleuchtstofflampen im elektronischen Hochfrequenzbetrieb verwirklichen. Diese haben den Vorteil, weder beim Start der Lampen noch während des Betriebs zu flackern. Sie bieten außerdem eine hohe Lichtausbeute und ein dem Sonnenlicht ähnliches Energiespektrum.

Bei dem eingesetzten graphischen Farbflachbildschirm und der beschriebene Durchleuchtung ist die Darbietung nur eine Frage der Auslegung der Ansteuerung, also der Software.

Die Darstellung in Gruppen oder Einzeldarbietung, zufallsgesteuerte Wiederholung, schneller Wechsel der Sehzeichen, bewegte Sehzeichen und weitere Varianten sind daher relativ einfach zu verwirklichen, auch kombiniert mit veränderbarem Kontrast und in unterschiedlichen Farben.

Für die simultane Trennung der Seheindrücke ist jedoch ein zusätzliches Element notwendig.

Um die gefundene Lösung der Aufgabe verständlich darstellen zu können, soll im folgenden zuerst kurz auf das Funktionsprinzip von Farbflachbildschirmen, speziell auf das beim Sehprüfgerät POLATEST E verwendete sogenannte "Aktivmatrixdisplay" oder "TFT-LCD", eingegangen werden.

Farbflachbildschirme bestehen aus einer Vielzahl von Flüssigkristallelementen, in denen der elektro-optische Effekt von flüssigen, anisotropen Kristallen zur Lichtsteuerung eingesetzt wird. Diese Flüssigkristalle können durch elektrische Felder in eine Vorzugsrichtung orientiert werden. Die anisotropen Eigenschaften dieser so orientierten Kristalle bewirken, daß polarisiertes Licht, je nach seiner Polarisationsrichtung geschwächt, völlig gesperrt oder durchgelassen wird.

Diese Zustände werden folgendermaßen verwirklicht: In der Flüssigkristallzelle sind Orientierungsschichten so angebracht, daß die Längsachsen der Moleküle, welche die linke und rechte Glasfläche berühren, im ausgeschalteten Zustand um 90° zueinander gedreht sind. Wegen des Bestrebens der Moleküle, sich in der flüssigkristallinen Schicht parallel zueinander auszurichten, dreht sich ihre Vorzugsrichtung zwischen den beiden Glasflächen um 90°. Die Flüssigkristalle ordnen sich an wie in einem verdrillten Band.

Die Polarisationsfilter sind so angeordnet, daß ihre Durchlaßrichtung mit der Vorzugsrichtung der Moleküllängsachsen an den beiden Glasflächen zusammenfällt. Das durchtretende polarisierte Licht läßt sich von den Molekülen des Flüssigkristalls leiten und folgt ihrer verdrillten Struktur so, daß seine Schwingungsebene nach Durchlaufen der Schicht an der anderen Begrenzungsfläche um insgesamt 90° gedreht worden ist. Da es damit parallel zum zweiten Polarisationsfilter orientiert ist, kann Licht ungehindert aus der Flüssigkristallzelle austreten. Die Flüssigkristallzelle ist lichtdurchlässig; sie erscheint hell.

Anders verhält es sich, wenn an den beiden Elektroden der Flüssigkristallzelle eine Spannung anliegt. In diesem Falle haben die flüssigkristallinen Moleküle die Tendenz, sich mit ihrer Längsachse in Feldrichtung zu drehen: Bei genügend großer Spannung stellen sie sich alle senkrecht zu den Glasflächen, außer in einem schmalen Übergangsbereich. Dadurch wird die Polarisationsebene des Lichtes beim Durchlaufen der Schicht nicht mehr gedreht - der zweite Polarisationsfilter wird zum undurchdringlichen Hindernis. Die Flüssigkristallzelle ist lichtundurchlässig; sie erscheint dunkel.

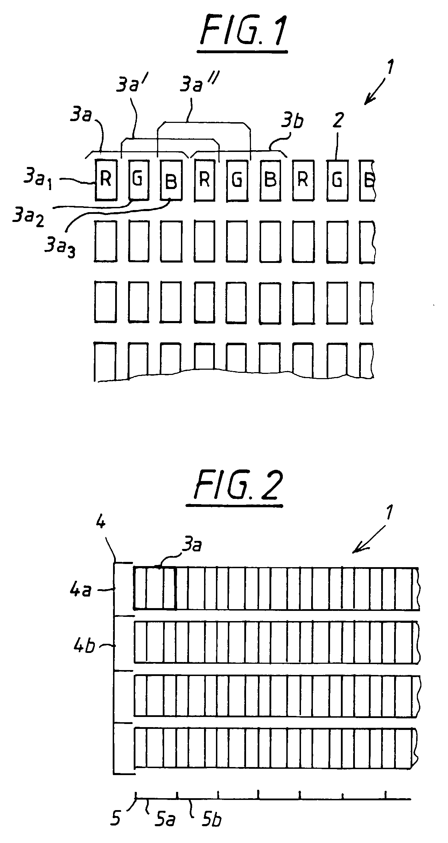

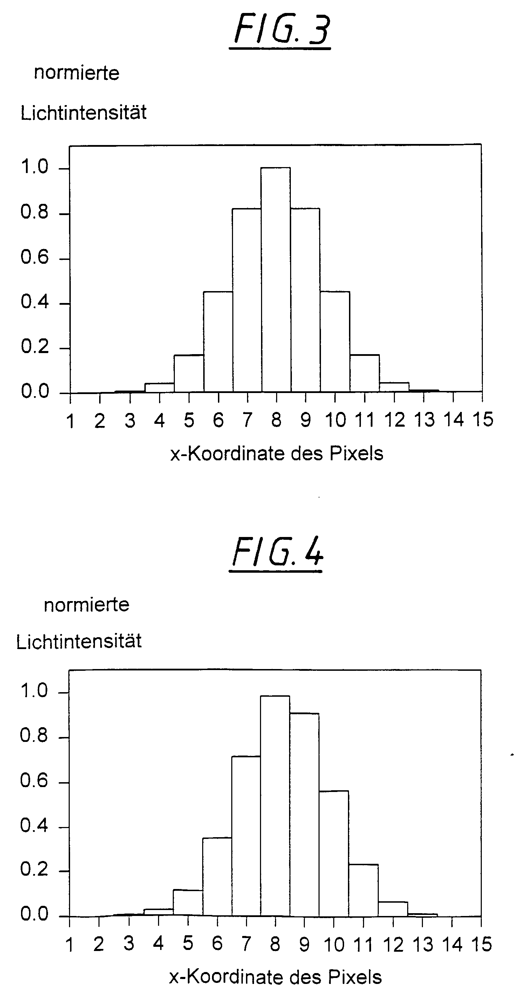

Viele derartigen Zellen nebeneinander angeordnet ergeben einen graphischen Farbflachbildschirm mit 640 x 480 = 307 200 Pixeln. Der Aufbau eines derartigen graphikfähigen Farbflachbildschirmes (1) ist in Figur 1 und 2 schematisch dargestellt.

Jedes Pixel (3a) ist wiederum aus roten, grünen und blauen Farbbereichen (bzw. Pixelteilen) (3a1, 3a2, 3a3) zusammengesetzt, die einzelnen wie ein rotes, grünes und blaues Lichtventil wirken. Sie lassen, je nach ihrer elektronischen Ansteuerung, mehr oder weniger rotes, grünes und blaues Licht durch.

Angesteuert werden diese durch ein elektronisches Schaltelement (Transistor), das direkt am Lichtventil angebracht ist. In einer Makrophotographie (Durchmesser = 1 mm) sind diese links in der oberen Ecke jedes Lichtventils zu erkennen. Die Leitungsbahnen zur elektronischen Steuerung sind dabei als dunkle Bänder ebenfalls sichtbar.

Die drei Farbbereiche (3a1, 3a2, 3a3) Rot/Grün/Blau zusammengenommen ergeben den Seheindruck eines farbigen Pixels (3a) in der Größe 0,33 mm x 0,33 mm. Aus vier Metern betrachtet weist jedes einzelne farbige Pixel (3a) einen Sehwinkel von 0,28 Winkelminuten auf. Da das Auflösungsvermögen des Auges stets schlechter als 0,5 Sehwinkelminuten ist, erzeugen die 307 200 farbigen Pixel (3a, 3b) des 16 x 21 cm großen Bildschirmes (1) ab einer Entfernung von 4 Metern also einen kontinuierlich erscheinenden Bildeindruck.

Für die simultane Darbietung der Sehzeichen zur Binokularprüfung wird ein Spezialfilter vor dem Farbflachbildschirm angebracht, der gleichzeitig, zeilenweise abwechselnd den Seheindruck für das rechte und linke Auge polarisiert. Dieser Spezialfilter muß genau auf die Elementstruktur des Bildschirmes justiert sein, um eine saubere Trennung zwischen den Bildern für das rechte und das linke Auge zu gewährleisten. Das Material, welches für diesen Spezialpolarisator verwendet wird, ist das gleiche, das im Polatest-Sehprüfgerät für die Random-Dot-Teste seit langem benutzt wird. Mit dieser Zusatzeinrichtung ist die simultane Darbietung der Sehzeichen mit positiver Polarisation verwirklicht.

Für beide Darbietungsarten - mit und ohne Trennung der Seheindrücke - erfolgt die Ansteuerung und Erzeugung der Teste mit modernster Computertechnologie, die den Anforderungen eines schnellen Bildaufbaues der Sehzeichen gerecht wird.

Das Herzstück dieser Ansteuerung ist in diesem speziellen Beispielfall ein CYRIX 486SLC Mikroprozessor, der mit 33 MHz Taktfrequenz arbeitet. Ein spezieller, auf den Flachbildschirm (1) abgestimmter Graphik-Controller ist für die Ansteuerung des LCD-Bildschirms (1) notwendig.

Beide Komponenten sind auf kleinstem Raum im neuesten Industriestandard, dem sogenannten PC/104-Format verwirklicht. Die Rechenleistung eines kompletten 486/33-MHz-Rechners ist hiermit auf 96 x 90 x 35 mm komprimiert.

Als Datenspeicher für die Sehzeichen werden vorzugsweise PCMCIA-Flashkarten eingesetzt. Diese haben gegenüber den normalerweise als Massenspeicher verwendeten Festplatten viele Vorteile:
- schnelle Zugriffszeiten,
- unempfindlicher, da keinerlei mechanisch bewegte Teile,
- Software Update durch einfachen Tausch der Karten,
- kleiner und
- geringerer Stromverbrauch.

Die Auswahl und Anwahl der Sehzeichen erfolgt durch eine Infrarot-Fernsteuerung.

Die eingangs gestellte Frage nach der Technologie für ein universell einsetzbares Sehprüfgerät ist durch die vorgestellte Kombination eines durchleuchteten elektronisch gesteuerten Farbflachbildschirmes (1) mit einem hochpräzis hergestellten Spezialpolarisator gelöst worden. Brillenglasbestimmung und Sehleistungsprüfungen sind damit uneingeschränkt durchführbar.

Der Leistungsumfang des Sehprüfgerätes POLATEST E ist zusammenfassend im folgenden kurz dargestellt.

Das 16 x 21 cm große Testfeld (1), in dem die Sehzeichen dargeboten werden, weist eine Leuchtdichte von etwas über 300 cd/m² auf und hat eine sonnenlichtähnliche Lichtfarbe mit der Farbtemperatur 4.000° K. Der Kontrast von dunklen Sehzeichen auf hellem Testfeld beträgt 97 %. Das Prüffeld (1) und die Sehzeichen können in ihrer Helligkeit unabhängig voneinander in 32 Stufen geändert werden. Dadurch ist z.B. eine inverse Darstellung der Sehzeichen (hell auf dunklem Grund) möglich.

Der Kontrast der Sehzeichen ist in 32 Graustufen einstellbar, das Prüffeld und die Sehzeichen sind in Farbe (32 768 Farben) und Helligkeit variierbar, so daß sich neue Dimensionen der Sehprüfung eröffnen.

Unterschiedliche Arten von Sehzeichen - Buchstaben, Ziffern, Landoltringe, Snellen-E und Kindersehzeichen - können angewählt und in unterschiedlichen Größen für einen Bereich der Visusanforderungen zwischen 0,05 und 2,0 dargeboten werden. Bei der Darbietungsart kann zwischen Gruppendarstellung (eine oder mehrere Zeilen unterschiedlicher Visusanforderung) und Einzeldarbietung gewählt werden. Der Bildwechsel erfolgt innerhalb von 0,5 Sekunden. Die Reihenfolge der Bilder kann individuell eingestellt werden, z.B. für die Brillenglasbestimmung, bei der eine festgelegte Reihenfolge der Sehzeichen vorteilhaft ist. Zahlreiche Sonderteste für die verschiedenartigsten Funktionsprüfungen sind vorhanden.

Die Sehschärfebestimmung ist normgerecht mit Landoltringen und Buchstaben möglich. Eine Besonderheit ist in der Möglichkeit zu sehen, zwischen Gruppendarbietung und Einzeldarbietung mit vorgegebener und/oder zufallsgesteuerter Reihenfolge der Sehzeichen wählen zu können.

Die binokulare Brillenglasbestimmung erfolgt bei simultaner positiver Polarisationstrennung der Seheindrücke. Mit dieser Technik können alle gebräuchlichen Systeme der binokularen Funktionsprüfung verwirklicht werden. Derzeit sind die Sehzeichen der Meß- und Korrektionsmethodik nach H.J. Haase (MKH), Abgleichteste und Elemente der graphischen Analyse vorhanden.

Speziell sei hier erwähnt, daß erstmals die berechtigte Forderung nach einem gleichgroßen Netzhautbild für die Sehzeichen bei unterschiedlichen Prüfentfernungen verwirklicht werden konnte. Bisher hatten beim Polatest-Sehprüfgerät die Sehzeichen nur in 5,5 m Prüfentfernung die ursprünglich erarbeiteten Abmessungen auf der Netzhaut.

Dieses Sehprüfgerät ermöglicht eine Vielzahl weiterer Anwendungen. Es ist universell einsetzbar und eröffnet daher ein weites Feld für interdisziplinäre Zusammenarbeit bei der Weiterentwicklung der Sehleistungsprüfung. Am POLATEST E-Sehtestgerät selbst ändern derartige Weiterentwicklungen nichts. Nur die Software und/oder die Display-Ansteuerung muß entsprechend ergänzt werden, will man das erfindungsgemäße Verfahren einsetzen.

Die Erzeugung der Stereobildpaare erfolgt bei dem erfindungsgemäßen Sehtestgerät dadurch, daß das Teilbild für das rechte Auge über die ungeradzahligen Zeichen eines TFT-Displays und das Teilbild für das linke Auge über die geradzahligen Zeilen (4a, 4b) dargestellt wird. Für den Betrachter werden die Teilbilder für das rechte und linke Auge durch einen Streifenpolarisator vor dem TFT-Display und eine entsprechend polarisierte Brille getrennt.

Zur Erzeugung des räumlichen Seheindrucks werden diejenigen Bildanteile, die sich räumlich aus dem Display abheben sollen, in einem der beiden Teilbilder um eine bestimmten Betrag seitlich (querdisparat) versetzt.

Der zur Durchführung des ersten erfinderischen Verfahrens benötigte Farbbildschirm muß eine Ansteuerungseinrichtung aufweisen, bei der jedes Pixel vorzugsweise aus drei nebeneinanderliegenden Farbrechtecken (z.B. mit den drei Grundfarben Rot, Grün und Blau) aufgebaut ist.

Die Ansteuerungseinrichtung des Farbbildschirms muß so aufgebaut sein, daß die farbigen Pixelteile auf dem Farbbildschirm unabhängig voneinander angesteuert werden können.

Das erste Verfahren beruht auf der Tatsache, daß sich jedes Pixel (Figur 1 mit 3a) des TFT-Displays aus drei schmalen nebeneinander liegenden farbigen Rechtecken (3a1, 3a2, 3a3), die in den Grundfarben rot, grün und blau leuchten, aufbaut. Jedes dieser Rechtecke (3a1, 3a2, 3a3) ist etwa 0,11 mm breit und 0,33 mm hoch.

Da zur Augenprüfung nur nahezu unbunte Stereobilder benötigt werden, kann die Schrittweite der Querversetzung von Bildbereichen dadurch verkleinert werden, daß Bildpunkte nicht um eine ganzzahlige Anzahl von Pixeln versetzt werden (z.B. durch eine Versetzung des RGB-Farbtripels bei 3a um eine Pixelbreite nach 3b), sondern in Schritten von 1/3-Pixel. Soll z.B. ein Bildpunkt bei 3a um 1/3-Pixel verschoben werden, so löscht man das links befindliche rote Farbrechteck (3a1) aus und fügt auf der rechten Seite ein rotes Farbrechteck (3b1) an. Dadurch wird aus dem RBG-Pixel (3a) ein BGR-Pixel (3a').

Die Kennzeichen des neuen Verfahrens sind,
a) daß Bildpunkte auf dem Bildschirm um weniger als eine Pixelbreite verschoben werden können, bzw.
b) daß der minimale realisierbare Stereowinkel nicht durch die Pixelbreite begrenzt ist, sondern auf ein Drittel des herkömmlichen Wertes verringert werden kann, bzw.
c) daß Bildpunkte auf dem Bildschirm in Schritten von einem Drittel der Pixelbreite verschoben werden können.

Mit dem ersten erfindungsgemäßen neuen Verfahren ist es also möglich, die minimale Verschiebung von Bildanteilen um den Faktor 3 gegenüber dem derzeitigen Wert zu verringern. Mit der erfindungsgemäßen Methode kann aus 5 m Distanz mit dem Polatest E also eine minimale Querdisparation von 4,54 Bogensekunden erzielt werden.

Auch durch ein weiteres Verfahren kann man die Positioniergenauigkeit von Bildbereichen auf einem Bildschirm verbessern.

Der zur Durchführung des zweiten erfinderischen Verfahrens benötigte Bildschirm muß eine Ansteuerungseinrichtung aufweisen, die die Erzeugung einer Schwerpunktverschiebung der Lichtverteilung eines darzustellenden Bildbereichs ermöglicht.

Bei diesem Verfahren sollten die Sehobjekte keine einzelnen Punkte sondern eine aus mehr als einem Pixel bestehende Lichtverteilung sein. In Figur 3 wurde zur Veranschaulichung angenommen, daß dem linke Auge ein schmaler, senkrechter Lichtbalken angeboten werden soll, der im horizontalen Querschnitt eine gaussförmige Lichtverteilung hat. Auf dem Bildschirm wird diese Lichtverteilung durch die Lichtintensitäten an den durch die Pixel vorgegebenen Stützstellen angenähert. Das visuelle System kann aufgrund seiner integrierenden Fähigkeit sehr genau die Lage des Schwerpunktes dieser Lichtverteilung lokalisieren. Um jetzt einen seitlich versetzten Lichtbalken für das rechte Auge zu generieren, muß die Ansteuerungseinheit die Lichtintensitäten einer Gausskurve berechnen, deren Lichtschwerpunkt entsprechend verschoben ist, und als Teilbild für das rechte Auge anzeigen. Mit diesem Verfahren kann man beliebig kleine Bildversetzungen erzeugen, die durch die Fähigkeit des visuellen Systems zur Auffindung des Lichtschwerpunkts entsprechend lokalisiert werden und bei einer haploskopischen Darbietung in einen entsprechenden räumlichen Seheindruck umgesetzt werden.

In den Figuren 3 und 4 ist dieses Verfahren für eine Verschiebung der gaussförmigen Lichtkurve entlang einer Zeile dargestellt, wobei in der Figur 3 die Lichtintensität für das rechte Auge und in der Figur 4 die Lichtintensität für das linke Auge dargestellt ist. Auf der vertikalen Achse ist dabei die Lichtintensität und auf der waagrechten Achse die Position eines Pixels aufgetragen, wobei ein Skalenteil mit der Größe eines Pixels übereinstimmt. In Figur 4 ist die Verteilung der Lichtintensität im Vergleich zur Figur 3 versetzt, so daß der Lichtschwerpunkt um 1/3 der Pixelbreite nach rechts verschoben ist.

Wie diese beiden Figuren 3 und 4 belegen, kann man bei einem Sehzeichen allein durch eine Veränderung der Lichtintensität an den durch die Position der Pixel vorgegebenen Stützstellen eine Verlagerung des Lichtschwerpunktes und damit eine seitliche Verschiebung des Bereichs bewirken.

Auch dieses zweite Verfahren verbessert das Meßverfahren zur Bestimmung des Stereogrenzwinkels entweder allein oder in Kombination mit dem ersten Verfahren.

Das zweite Verfahren ist nicht auf Farbbildschirme beschränkt, sondern es ist auch auf einem schwarz/weiß Bildschirm einsetzbar.

Die Positioniergenauigkeit von Bildbestandteilen wird mit dem zweiten Verfahren nicht nur in horizontaler Richtung sondern auch in beliebigen anderen Richtungen wesentlich erhöht. Für die Prüfung des Stereosehens ist allerdings nur die Verbesserung der horizontalen Auflösung von Bedeutung.

Das erste Verfahren ist gut geeignet, um Sehzeichen, die aus kleinen Punkten oder Linien bestehen, wie z.B. Random-Dot-Stereogramme, 3-dimensional darzustellen. Bei diesem Verfahren ist die Schrittweite aber auf eine Bildverschiebung von 1/3 der Pixelbreite limitiert.

Das zweite Verfahren ist vorzugsweise für etwas breitere Lichtverteilungen geeignet, hat aber den Vorteil, daß sich eine praktisch beliebig genaue Positioniergenauigkeit erreichen läßt und eine Verschiebung in alle Richtungen möglich ist.

Die erfindungsgemäßen Verfahren sind nicht auf TFT-Displays (1) beschränkt, sondern können auch bei anderen Sehtestgeräten, die mit herkömmlichen Fernsehmonitoren arbeiten, angewandt werden. Monitore, die die Sony-Triniton Bildröhre verwenden, haben eine dem TFT-Display ähnliche RGB-Streifenmaske, bei sich jeder Bildpunkt aus drei nebeneinander liegenden Farbrechtecken zusammensetzt. Auch Eizo Monitore verwenden einen ähnlichen Aufbau der Pixel. Auch auf einfache Farbfernsehmonitore, die eine hexagonale Bildpunktmatrix aufweisen, lassen sich diese Verfahren anwenden.

Wenn über einem Fernsehmonitor kein Streifenpolarisator angebracht werden kann, müssen allerdings andere Trennerverfahren zur haploskopischen Darbietung, wie z.B. eine alternierende Bilddarbietung für das rechte und linke Auge mittels LCD-Shutterbrillen, eingesetzt werden.

## Patentansprüche

1. Verfahren für die Bilderzeugung auf einem Farbbildschirm mit einer Ansteuerungseinrichtung, wobei jedem Pixel (3a) drei beieinander angeordnete, unterschiedliche Farben emmitierenden Farbbereiche (3a1, 3a2, 3a3) zugeordnet sind und die Pixel (3a, 3b) in Zeilen (4a, 4b) und/oder Spalten (5a, 5b) angeordnet sind, dadurch gekennzeichnet, daß durch die Ansteuerungseinrichtung des Farbbildschirms (1) ein sichtbarer Bildpunkt (3a') auf dem Farbbildschirm (1) durch drei nebeneinander liegende Farbbereiche (3a2, 3a3, 3b1) unterschiedlicher, nebeneinander liegender Pixel (3a, 3b) erzeugt wird.

2. Verfahren für die Bilderzeugung auf einem Bildschirm mit einer Ansteuerungseinrichtung, wobei die Pixel (3a, 3b) in Zeilen (4a, 4b) und/oder Spalten (5a, 5b) angeordnet sind, dadurch gekennzeichnet, daß ein oder mehrere darzustellende Bildbereiche aus mehr als einem Pixel (3a, 3b) gebildet werden, wobei die darzustellenden Bildbereiche einen Schwerpunkt der Lichtverteilung besitzen, und daß für eine Bildbereichsverschiebung auf dem Bildschirm die verschiedenen Pixel (3a, 3b) so durch die Ansteuerungseinrichtung angesteuert werden, daß die sich eine vorgegebene Schwerpunktverschiebung des darzustellenden Bildbereichs ergibt.

3. Verfahren nach Anspruch 2, dadurch gekennzeichnet, daß die Gesamtintensität der verschobenen Lichtverteilung gleich der Gesamtintensität der unverschobenen Lichtverteilung ist.

4. Verfahren nach Anspruch 1, dadurch gekennzeichnet, daß das Verfahren nach Anspruch 1 mit dem Verfahren nach Anspruch 2 kombiniert wird.

5. Verfahren nach einem der Ansprüche 1-4, dadurch gekennzeichnet, daß jedem Auge ein separates Teilbild dargeboten wird.

6. Verfahren nach einem der Ansprüche 1-5, dadurch gekennzeichnet, daß der Bildschirm in einem Sehtestgerät verwendet wird.

7. Verfahren nach Anspruch 6, dadurch gekennzeichnet, daß der oder die darzustellenden Bildbereiche Sehzeichen sind.

8. Verfahren nach einem der Ansprüche 6 oder 7, dadurch gekennzeichnet, daß das Sehtestgerät zur Messung der Wahrnehmungsschwelle des Stereosehens gemäß DIN 5340 des visuellen Systems einer zu untersuchenden Person verwendet wird, wobei in einem vorab festgelegten Abstand definierte Bildbereiche dargeboten werden.

9. Farbbildschirm mit einer Ansteuerungseinrichtung, wobei einem Pixel (3a, 3b) drei beieinander angeordnete, unterschiedliche Farben emmitierenden Farbbereiche (3a1, 3a2, 3a3) zugeordnet sind und die Pixel (3a, 3b) in Zeilen (4a, 4b) und/oder Spalten (5a, 5b) angeordnet sind, dadurch gekennzeichnet, daß die Ansteuerungseinrichtung des Farbbildschirms (1) so aufgebaut ist, daß ein sichtbarer Bildpunkt (3a') auf dem Farbbildschirm (1) durch drei nebeneinander liegende Farbbereiche (3a2, 3a3, 3b1) unterschiedlicher, nebeneinander liegender Pixel (3a, 3b) erzeugt wird und/oder ein oder mehrere darzustellende Bildbereiche aus mehr als einem Pixel (3a, 3b) gebildet werden, wobei die darzustellenden Bildbereiche einen Schwerpunkt der Lichtverteilung besitzen, und für eine Bildbereichsverschiebung eine Schwerpunktverschiebung des darzustellenden Bildbereichs erfolgt.

10. Farbbildschirm nach Anspruch 9, dadurch gekennzeichnet, daß der Farbbildschirm (1) ein TFT-Display ist.

11. Farbbildschirm nach einem der Ansprüche 9 oder 10, dadurch gekennzeichnet, daß über dem Farbbildschirm (1) ein Streifenpolarisator angebracht ist.

12. Farbbildschirm nach einem der Ansprüche 9-11, dadurch gekennzeichnet, daß ein Pixel (3a) aus drei nebeneinanderliegenden Farbrechtecken (3a1, 3a2, 3a3) mit den drei Grundfarben Rot, Grün und Blau aufgebaut ist.

13. Farbbildschirm nach einem der Ansprüche 9-12, dadurch gekennzeichnet, daß eine Lambda-Viertel-Folie vor oder nach der Polarisationsfolie auf dem Bildschirm (1) angebracht ist.

14. Farbbildschirm nach einem der Ansprüche 9-13, dadurch gekennzeichnet, daß der Farbbildschirm (1) Teil eines Sehtestgerätes ist.

15. Farbbildschirm nach Anspruch 14, dadurch gekennzeichnet, daß auf dem Bildschirm (1) des Sehtestgeräts auch farbige Sehzeichen darstellbar sind.

16. Farbbildschirm nach einem der Ansprüche 14 oder 15, dadurch gekennzeichnet, daß auf dem Display (1) des Sehtestgeräts auch bewegte Sehzeichen darstellbar sind.

17. Farbbildschirm nach einem der Ansprüche 14-16, dadurch gekennzeichnet, daß der Farbbildschirm Teil eines Sehtestgerät ist, bei welchem zur Messung einer räumlichen Wahrnehmungsschwelle des visuellen Systems einer zu untersuchenden Person in einem vorab festgelegten Abstand Sehzeichen dargeboten werden.
